# EUROPEAN PATENT APPLICATION

(11) **EP 4 811 419 A2**
(43) Date of publication of application: **23.09.2026**
(21) Application number: 26163594.0
(22) Date of filing: 10.03.2026
(51) Int. Cl.: H01J 49/00, G01N 27/623, G01N 33/68

(54) **MASS SPECTROMETRIC METHOD AND MASS SPECTROMETER SYSTEM FOR DETECTING AN ANALYTE FROM A BIOLOGICAL SAMPLE**

(30) Priority: 19.03.2025 WO PCT/EP2025/057450
(71) Applicant: Roche Diagnostics GmbH, 68305 Mannheim (DE)
(72) Inventor: The designation of the inventor has not yet been filed
(74) Representative: Schach, Denise Kristine

(57) **Abstract**

A mass spectrometric method for detecting an analyte from a biological sample, the method comprising: Collecting with a transfer device a pre-processed sample comprising analyte-loaded magnetic microparticles to which the analyte is captured; Transferring with the transfer device the pre-processed sample to a substrate; Subjecting the pre-processed sample on the substrate to Direct Analysis in Real Time (DART) ionization to generate ionized analyte molecules; Detecting the ionized analyte molecules using a mass spectrometer.

## Description

### Field of the Invention

The present invention relates to a mass spectrometric method and a mass spectrometer system for detecting an analyte from a biological sample as well as a computer-program product for carrying out the method in the field of In Vitro Diagnostics.

### Background of the Invention

Mass spectrometry is widely used for the qualitative and quantitative analysis of chemical substances ranging from small molecules to macromolecules. Often, mass spectrometry is combined with chromatographic techniques, particularly gas and liquid chromatography such as high-performance liquid chromatography. In that case, the analyzed molecule of interest is separated chromatographically and is individually subjected to mass spectrometric analysis. While separation via chromatography offers advantages in terms of pre-enrichment, it has inherent problems regarding the very limited time span for the measurement of the enriched analyte as a peak. Further, this combination has inherent problems of analyte-surface contact and the need for stationary phases and liquid solvents. Chromatography is often cumbersome to use in clinical diagnostics, which seeks to achieve a high throughput in sample analysis, as the mechanical parts underlie inherent stress and the stationary phases need to be exchanged on a regular basis due to deterioration.

When avoiding chromatography, achieving a high degree of sensitivity and selectivity is often challenging, specifically when using direct infusion techniques. Moreover, most techniques require providing liquid samples after extraction. Using solutions for elution may also cause a dilution effect which makes high sensitivity difficult to achieve.

### Summary of the Invention

It is therefore desirable to provide an approach that allows increased sensitivity and avoiding contamination.

Moreover, there is a need for a high degree of versatility with respect to the form of samples being provided. In addition, there is a need for a simplified mass spectrometric method, specifically one that is user-friendly and reduces the efforts required in sample preparation. Specifically, it is desirable to provide an approach that reduces the intensive maintenance requirements. Moreover, it is desirable to provide a mass spectrometric workflow that avoids time-consuming sample preparation steps. There is also a need for a method that can overcome the temporal limitations dictated by the elution of an enriched analyte as a peak from a stationary phase, which often limits the time span available for detection.

Moreover, there is a need for a method that improves sustainability.

Additionally, there is a need for an analytical process that eliminates the matrix effects and ion suppression commonly encountered when analyzing complex biological matrices like serum.

Finally, there is a need for a system that provides standardized procedures to reduce user-dependent factors and manual steps, thereby ensuring higher reproducibility and reliability in clinical diagnostics.

At least one of these desired improvements can be achieved by the aspects of this disclosure, specifically the subject matter defined by the independent claims. Further advantageous effects and/or improvements being mentioned above or even exceeding such desired improvements are achieved by the specific embodiments, i.e. the corresponding subject matter covered by the dependent claims.

According to an aspect that may be considered a first aspect, a mass spectrometric method for detecting an analyte from a biological sample comprises: collecting with a transfer device a pre-processed sample comprising analyte-loaded magnetic microparticles to which the analyte is captured; transferring with the transfer device the pre-processed sample to a substrate; subjecting the pre-processed sample on the substrate to Direct Analysis in Real Time (DART) ionization to generate ionized analyte molecules; detecting the ionized analyte molecules using a mass spectrometer.

The mass spectrometric method may provide a robust method that allows increased sensitivity and decrease or even avoid contamination. The mass spectrometric method may further improve the degree of versatility with respect to the form of samples being provided. The mass spectrometric method may further refer to a simplified mass spectrometric method, specifically one that is user-friendly and reduces the efforts required in sample preparation. Using DART ionization, the method reduces the intensive maintenance requirements related to chromatography. For the same reason, the mass spectrometric workflow may avoid time-consuming sample preparation steps, may overcome the temporal limitations dictated by the elution of an enriched analyte as a peak from a stationary phase, and may improve sustainability as the amount of solvents may be reduced compared to prior art mass spectrometric methods and systems and due to bypassing the chromatography that requires extensive amounts of solvents at high purity grade. Additionally, the mass spectrometric method may eliminate the matrix effects and ion suppression commonly encountered when analyzing complex biological matrices like serum. Finally, the mass spectrometric method may provide standardized procedures to reduce user-dependent factors and manual steps, thereby ensuring higher reproducibility and reliability in clinical diagnostics. By enabling the direct analysis of analytes bound to solid supports such as magnetic microparticles, the invention may streamline clinical workflows, may reduce the complexity of sample preparation, and may avoid dilution effects inherent in traditional extraction protocols.

The technical advantage of the method of the first aspect may comprise the direct coupling of solid-phase capture with ambient ionization. By using the magnetic microparticles as a carrier for the analyte in the DART analysis, the workflow surrenders the need for an additional elution step, which typically involves organic solvents and leads to sample dilution. This results in higher sensitivity and a simplified, faster analytical process suitable for high-throughput clinical environments. The technical effect is the efficient thermal desorption and ionization of analytes directly from the surface of the microparticles, where the thermal conductivity of the particles or the substrate facilitates energy transfer from the gas stream to the target molecules.

The term **"biological sample"** may comprise a sample from a patient, and it may comprise at least one of: fluid samples such as blood, serum, plasma, synovial fluid, spinal fluid, urine, saliva, and lymphatic fluid, or solid samples such as dried blood spots and tissue extracts; a part or piece of a tissue, organ.

The term **"analyte"** may comprise "an analyte of interest", namely a chemical species to be analyzed via mass spectrometry, which may comprise a molecule present in a living organism, including but not limited to nucleic acids, amino acids, peptides, proteins, metabolites, hormones and/or substances internalized by the organism such as (therapeutic) drugs.

The term **"transfer device"** may comprise a device for adding and/or removing fluids and/or solids. A transfer device may comprise at least one of: a pipetting device, for example based on suction force to receive liquids and using (disposable) pipette tips; a liquid handling unit; a fluid transport device; a solid transfer device that may comprise and/or may be operated with a spatula-like tool and/or a spoon-like tool. The transfer device may correspond to a transfer tool. The transfer device may specifically comprise or consist of non-metallic elements or may have at least non-metallic elements, specifically replaceable non-metallic elements. The term "transfer device" may comprise in all cases to a single-use device and/or to a device that is operated in combination with single-use elements and/or that comprises single-use elements.

The term **"detecting an analyte"** may comprise the mere qualitative detection of presence or the quantitative determination of the level of the analyte, including absolute or relative amounts.

The term **"magnetic microparticles"** may comprise spherical and/or irregularly shaped particles in a range of 10 nm to 10 mm comprising inorganic and/or organic material that is magnetic and/or paramagnetic. The term "magnetic microparticles" may be understood as magnetic beads and/or magneto-beads.

The term **"captured"** may comprise the analyte being desorbed, adsorbed and/or bound (specifically bound) to the solid surface of the microparticles. The process may be denoted "magnetobead-capturing".

The term **"analyte-loaded magnetic microparticles"** may therefore refer to magnetic microparticles that are loaded with the analyte of interest. Specifically, the magnetic microparticles may be provided with analyte-specific antibodies that specifically bind the analyte of interest. The term "analyte-loaded magnetic microparticles" may comprise "analyte-enriched magnetic microparticles". The term "analyte-loaded magnetic microparticles" maybe understood a pre-processed sample.

The **"substrate"** may comprise a support element configured to receive a sample (here the analyte-loaded magnetic microparticles) that is stationary and/or permanently placed in a mass spectrometer system that is configured to carry out the method. The substrate may be understood as the solid and/or permeable support on which the sample and/or pre-processed sample is deposited for Direct Analysis in Real Time ionization. While a metallic substrate, specifically in the form of a metallic mesh, metallic grid, or metallic plate with openings, provides excellent thermal conductivity and may allow a heated gas stream to pass through the sample, the substrate may alternatively comprise other non-metallic materials. Specifically, the substrate may comprise thermostable materials, specifically a synthetic thermostable material, a ceramic material, a glass-based material (e.g. porous ceramic or glass mesh) or a composite material possessing sufficient thermal and chemical stability to withstand the Direct Analysis in Real Time ionization process. Furthermore, the substrate may be embodied as a sample card, a strip, a multi-well plate, a wire, or a fibrous matrix. The substrate may specifically be configured to support the magnetic microparticles such that they are presented to the Direct Analysis in Real Time ion source in a manner that allows for efficient thermal desorption of the captured analyte into the gas phase. In all cases, the substrate may have the form of a mesh, a grid, or a plate with openings.

The transfer device is distinct from the substrate and therefore, a new handling system is provided specifically being configured to enable automated, high-sensitivity diagnostics without the need for IMS separation. In other words, the transfer device and the substrate are two separate entities.

The step of transferring may be considered a non-magnetic displacement of the microparticles from the transfer device to the substrate. The non-magnetic displacement may be considered a mechanical and/or physical movement of the analyte-loaded beads that does not rely on magnetic forces for the final deposition onto the ionization surface. In traditional workflows, magnetic beads are often moved and/or immobilized using magnetic rods and/or external fields during the entire process, including the measurement phase. In contrast, this specific method may involve capturing the analyte using magnetic microparticles but then utilizing a transfer device-such as a pipette tip, spatula, or spoon-to physically move those beads and "place" and/or "eject" them onto the substrate. Specifically, the step of transferring the pre-processed sample to the substrate is not performed under the impact of an external and/or additional magnetic field. In other words, the step of transferring the pre-processed sample to the substrate is not realized via a magnetic force / magnetic field (neglecting any magnetic field that might be caused by the magnetization of the microparticle itself).

This non-magnetic displacement provides several key technical interpretations and advantages, namely, instead of a magnet "pulling" the beads onto a surface, the transfer device (e.g., a pipette) displaces the suspension or solid particles via suction, air displacement and/or mechanical contact. Further, it allows the substrate to remain the "sole contact point" for the microparticles during the actual DART ionization step, as no magnetic probes need to be present in the ionization zone. By removing the requirement for magnetic immobilization during DART, the system may avoid potential interference between the DART gas stream and magnetic handling hardware, ensuring the gas can flow freely through the mesh. This approach may leverage standard clinical automation, such as liquid handling stations, which are designed for the non-magnetic displacement of fluids and suspensions using disposable tips. The technical effect may be provided in that the microparticles are deposited in a controlled, localized manner on the substrate (such as a mesh or grid), where they are then held by gravity or surface tension rather than active magnetic force, preparing them for efficient thermal desorption by the DART ion source.

The method may combine a magnetic bead based and/or a magnetically based sample preparation with a subsequent non-magnetic transfer step.

It is noted that one advantage of the present application may be the reduction of a DART gas stream and/or exchange of an expensive gas with a cheaper gas. In some cases, expensive gases (such as helium) which are often used as a DART gas stream may be replaced by cheaper alternatives such as nitrogen and/or air.

The mass spectrometric method may comprise the additional step of, prior to the step of collecting, providing the pre-processed sample comprising a suspension. The pre-processed sample may specifically correspond to a suspension.

The technical advantage of at least some of these features, specifically of using synthetic and/or disposable tips may comprise the prevention of cross-contamination and carry-over between different biological samples. The technical effect is the reliable volumetric transfer of the captured analyte to the ionization substrate without memory effects or the need for intensive maintenance and cleaning cycles between samples.

The term **"suspension"** may comprise a mixture in which magnetic microparticles are dispersed throughout a liquid phase, such as a biological sample or a derivative thereof.

The transfer device may comprise a non-metallic tip and/or a synthetic tip configured to receive at least a portion of the suspension, more specifically a disposable tip, a polymer and/or plastic tip and/or a pipette tip. Alternatively, the transfer device may comprise other materials such as a metal needle and the transfer device may resist aggressive liquids typically used to clean diagnostic and/or clinical instruments. In all cases, the transfer device or elements thereof may be disposable, i.e. simple to be replaced, not permanently attached and configured for single use.

The term **"synthetic tip"** may be understood as a consumable part made from polymer materials, such as a disposable pipette tip, designed for fluid handling and aspirating or dispensing specific volumes.

The step of collecting the pre-processed sample may comprise aspirating at least a portion of the suspension. The steps of collecting and/or transferring with the transfer device the pre-processed sample may comprise collecting and/or transferring at least a portion of the suspension onto the substrate via a displacement step, preferably using a synthetic tip, specifically via a non-magnetic displacement step and/or using a disposable tip and more specifically using a pipette tip.

The substrate may comprise: a metallic substrate, specifically a permeable and/or porous metallic substrate, specifically at least one of: a metallic mesh; a metallic grid; a metallic plate with openings.

The technical advantage of at least some of these features may be that a mesh structure allows the (heated) gas stream to pass through the substrate. The technical effect is an improved interaction between the metastable gas and the analytes on the microparticles, leading to more efficient desorption and higher signal intensity due to reduced physical blockage of the gas stream compared to a non-permeable surface.

The substrate may comprise: a non-metallic substrate, specifically a permeable and/or porous non-metallic substrate, specifically at least one of: a non-metallic mesh; a non-metallic grid; a non-metallic plate with openings.

The term **"metallic substrate"** may be understood as a solid support comprising and/or being made of metal that serves as the platform for DART ionization.

The term **"permeable"** may be understood as a structure allowing the passage of gas and/or liquids, such as a metallic mesh or grid with a plurality of openings, channels and/or holes.

In a specific embodiment, the step of transferring may specifically be considered a non-magnetic displacement of the microparticles from the pipette tip onto the metallic substrate, such as the metallic mesh prior to analysis.

The mass spectrometric method may further comprise: prior to the step of transferring the pre-processed sample to the substrate, a step of providing an un-used metallic substrate; and/or as a final method step, removing the substrate after single-use.

The technical advantage of at least some of these features may comprise the absolute prevention of memory effects and/or contamination from previous measurements, which is critical for high-sensitivity diagnostic accuracy. The technical effect is ensuring a pristine background for every sample, maintaining the integrity of trace analysis in clinical settings.

The term **"single-use"** as used herein for the substrate and/or the transfer device and/or elements thereof may be understood as a consumable strategy where the component/element is used for one sample analysis and then discarded and/or removed from the system. Therefore, a single-use a disposable and/or a consumable element may be structurally provided with means for temporal attachment. A single-use element, a disposable element and/or a consumable element may be easily replaced, removed and/or added, sometimes with tools but preferably without the need for additional tools.

The term **"un-used"** may relate to a substrate that is free from previous sample exposure, residual molecules, or environmental contamination.

The mass spectrometric method may further comprise a step of sample pre-processing to obtain the pre-processed sample. Specifically, the sample pre-processing may comprise the following steps: providing a mix comprising: the biological sample and/or a derivative thereof comprising the analyte and the mix comprising the magnetic microparticles that are surface functionalized to selectively bind the analyte from the biological sample or the derivative thereof; incubating the mix to obtain the analyte-loaded magnetic microparticles. More specifically, the sample pre-processing may comprise a step of washing the analyte-loaded magnetic microparticles.

The technical advantage may comprise the selective enrichment of the analyte from complex biological matrices while simultaneously removing interfering components like proteins and/or salts during the washing step. The technical effect may be the significant reduction of matrix effects and ion suppression, leading to cleaner ionization and more accurate mass spectrometric quantification.

The term **"sample pre-processing"** may comprise one or more analytical steps aimed at removing or reducing interfering matrix components and enriching analytes of interest.

The term **"surface functionalized"** may comprise the state of modification of the microparticle surface with chemical groups, core-shell structures, and/or specific coatings configured to selectively bind the analyte of interest, such as antibodies and/or magnetic graphene oxide composites.

The term **"washing"** may be understood as the process of removing unwanted matrix components and/or un-specifically bound components and/or molecules from the bound analyte-particle complex while the analyte remains captured on the solid support.

The mass spectrometric method may further comprise: prior to the step of collecting, providing the pre-processed sample in a dried form; and/or drying the pre-processed sample, specifically drying the pre-processed sample before the step of collecting the pre-processed sample and/or drying the pre-processed sample on the substrate before the step of subjecting the pre-processed sample to Direct Analysis in Real Time ionization.

The technical advantage of at least some of these features may be that drying can stabilize the analyte on the microparticle surface and may prevent potential splashing and/or sputtering when the sample is exposed to the high-temperature gas stream. The technical effect may be a controlled and/or concentrated sample surface that can improve the reproducibility of the thermal desorption process.

The term **"dried form"** may comprise the sample state after the partial or after the complete removal of the liquid solvent, for example via evaporation, to result in a solid residue and/or (quasi) dry powder.

The term **"drying"** may comprise the process of removing liquid by heating, gas flow, or ambient evaporation.

In a case when the pre-processed sample is provided in a dried form prior to the step of collecting, the transfer device may comprise a synthetic element configured to receive at least a portion of the pre-processed sample in a dried form. Specifically, the transfer device may comprise at least one of: a disposable spatula; a disposable spoon; a disposable spatula tip; a disposable spoon tip.

The technical advantage of at least some of these features, specifically of a method using such transfer devices, may comprise the ability to transfer analyte-loaded particles in scenarios where liquid handling is not feasible and/or where a completely dry workflow is preferred. The technical effect is the precise physical displacement of the purified solid sample onto the ionization mesh while maintaining the benefits of disposable consumables to prevent carry-over.

The term **"synthetic element"** may comprise a solid transfer tool comprising non-metallic materials such as plastic and/or polymers, specifically configured for handling dry solids.

The term **"spatula"** or **"spoon"** may comprise a mechanical tool for the physical displacement and collection of solid materials.

The substrate may be the sole contact point for the microparticles during the Direct Analysis in Real Time ionization step.

The technical advantage of at least some of these features, specifically of minimizing contact points, may comprise the reduction of potential loss of analyte to other surfaces and the prevention of contamination from system components. The technical effect may be that the microparticles are positioned such that they are fully and exclusively exposed to the ionizing gas stream while supported by the mesh, maximizing signal fidelity and desorption efficiency.

The term **"sole contact point"** may comprise a configuration where the analyte-loaded magnetic microparticles are supported exclusively by the substrate during ionization without other hardware interfaces, such as magnetic probes or rods. Specifically, a magnetic source which may be used in some cases to manipulate the magnetic microparticles may therefore never make physical contact with the sample.

The step of detecting may further comprise recording at least two pre-summed mass-resolved spectra and digitally summing the at least two pre-summed mass-resolved spectra to obtain a final mass-resolved spectrum representing an accumulation of the MS signal.

The technical advantage of at least some of these features may comprise a drastic improvement in the signal-to-noise ratio, as random background noise is statistically minimized while the consistent analyte signal is summed linearly. The technical effect is the ability to reveal clear peaks for trace analytes that would otherwise be indistinguishable from the background in a single-event measurement.

The term **"pre-summed mass-resolved spectra"** may comprise spectral data sets that have been initially accumulated by a first data processor integrated in the mass spectrometer.

The term **"digitally summing"** or **"digitally accumulating"** may comprise the process of adding multiple pre-summed spectra together to obtain a final spectrum, statistically vanishing background dark noise while summing real analyte events.

The step of detecting may be performed using the mass spectrometer being free of an Ion Mobility Spectrometry (IMS) separation.

The technical advantage of at least some of these features may comprise a reduction in hardware complexity, cost, and maintenance while supporting high sample throughput. The technical effect may also be that sufficient selectivity is provided by the combination of selective microparticle capture and high-resolution mass spectrometry, such that IMS is not required.

The term **"Ion Mobility Spectrometry"** may comprise a technique that separates gas-phase ions based on their size and shape in an electric field.

The term **"free of Ion Mobility Spectrometry separation"** may comprise a system configuration where the mass spectrometer operates without an active ion mobility separation module.

According to an aspect that may be considered a second aspect, a mass spectrometer system for detecting an analyte from a biological sample comprises: a transfer unit configured to collect with a transfer device a pre-processed sample comprising analyte-loaded magnetic microparticles to which the analyte is captured and the transfer unit configured to transfer with the transfer device the pre-processed sample to a substrate; a Direct Analysis in Real Time (DART) ion source configured to generate ionized analyte molecules from the analyte-loaded magnetic microparticles on the substrate; a mass spectrometer configured to detect the ionized analyte molecules.

The technical advantage of this system may comprise the integration of magnetic bead handling with ambient ionization mass spectrometry in a potentially fully automated platform. The technical effect may be a robust and/or fast diagnostic platform that minimizes manual labor and potential for human error in clinical laboratories.

The term **"transfer unit"** may comprise a robotic or automated module, such as a liquid handling station or automated arm, designed to move samples between prep stations and the mass spectrometer inlet.

The term **"Direct Analysis in Real Time ion source"** may comprise the apparatus providing a heated, electronically excited metastable gas stream at atmospheric pressure to desorb and ionize analytes.

The mass spectrometer system may further comprise a sample pre-processor configured to pre-process the biological sample or derivative thereof to obtain the analyte-loaded magnetic microparticles.

The technical advantage of at least some of these features may comprise the realization of a sample-in to result-out capability. The technical advantage of at least some of these features may further comprise the automated execution of the entire diagnostic cycle, which ensures highly reproducible sample preparation critical for clinical diagnostics.

The term **"sample pre-processor"** may be understood as an analytical module and/or station designed to execute automated processing steps such as pipetting, mixing, incubation, and magnetic separation.

The mass spectrometer system may comprise: a first data processor configured to record at least two pre-summed mass-resolved spectra, specifically, wherein the mass spectrometer further comprises: a controller module (also denoted "controller") comprising a second data processor configured to digitally sum the at least two pre-summed mass-resolved spectra to obtain a final mass-resolved spectrum.

The technical advantage of at least some of these features, specifically of this dual-processor architecture, may comprise the ability to perform summing on the fly during acquisition. The technical effect is that the system can monitor data quality in real-time and stop the measurement once a predefined precision threshold is reached, maximizing throughput.

The term **"first data processor"** may comprise an internal processor or digitizer integrated within the mass spectrometer architecture.

The term **"second data processor"** may comprise an additional and/or external and/or system-level controller configured for high-level data aggregation and summing.

The mass spectrometer system may further comprise a liquid handling station equipped with the transfer device, the transfer device comprising a disposable and/or synthetic pipette tip configured to aspirate a suspension comprising the analyte-loaded magnetic microparticles and displace said suspension onto the substrate.

The technical advantage of at least some of these features may comprise the use of standardized clinical automation for the precise delivery of magnetic beads to the ionization mesh. The technical effect is the repeatable deposition of the sample, which is a prerequisite for reliable quantitative analysis in in vitro diagnostics.

The term **"liquid handling station"** may be understood as an automated apparatus for pipetting fluids with high precision.

The term **"aspirate"** may be understood as the intake of fluid, and "displace" may be understood as the dispensing or ejection of fluid.

The mass spectrometer system may further comprise a sample receiving station equipped with the substrate, specifically a metallic substrate comprising a permeable and/or porous metallic substrate, more specifically at least one of: a metallic mesh; a metallic grid; a metallic plate with openings; or the substrate comprising a non-metallic substrate, specifically a ceramic substrate and/or a glass substrate, more specifically a porous ceramic or glass mesh.

The technical advantage of at least some of these features may comprise the standardized alignment of multi-sample cards, such as those with microtiter plate spacing. The technical effect may comprise the facilitation of high-throughput analysis where multiple samples can be analyzed in rapid succession using motorized rail systems.

The substrate, specifically the metallic mesh may comprise a high-throughput sample card configured for automated handling.

The term **"sample receiving station"** may comprise the mechanical interface and/or holder that may position the substrate in a moving trail of the ion source.

The term **"transfer unit"** may comprise a mechanical and/or automated system configured to move, displace, or deliver the sample between stations, and may comprise at least one of: a liquid handling assembly; a robotic transfer arm; an automated transfer assembly; a transfer assembly. This unit may be further embodied by specific structural components such as a multi-channel pipetting head, a motorized XYZ-stage, a pick-and-place robot, a conveyor-based transport system, a vacuum-assisted displacement module, or a centrifugal handling assembly. Where the transfer unit utilizes a transfer device, said device provides a broad range of structural examples including a synthetic pipette tip, a capillary, a needle, a syringe, a micro-nozzle, or a specialized solid-phase displacement tool designed for the physical collection and ejection of microparticles. The technical advantage of such a structural configuration is the precise, repeatable delivery of analyte-loaded microparticles to the substrate, while the technical effect is the enabling of a "sample-in to result-out" workflow that minimizes manual intervention and human error in high-throughput clinical diagnostics.

The term **"about" or "approximately"** in the context of numerical values or ranges may refer to a variation of 10% of the stated value, unless the specific context indicates a different tolerance.

Below are described some specific optional embodiments and features of the method according to the first aspect or embodiments thereof.

In some embodiments, the mass spectrometric method of the first aspect or an embodiment thereof may suited for detecting one or more analytes in a biological sample solution and may comprise the steps:
providing a constant stream of ionized sample molecules comprising the analyte from the biological sample and an analyte-specific internal standard including electrospray ionization (ESI) and/or nano-electrospray ionization (nano-ESI);
performing a spatial accumulation of the analyte and the analyte-specific internal standard and performing at least partially a first separation of the analyte and the analyte-specific internal standard from other components of the ionized sample molecules based on the size and/or shape of the ionized molecules;
performing at least partially a second separation of the analyte and the analyte-specific internal standard from remained other components of the ionized sample molecules based on the mass to charge ratio of the ionized molecules;
fragmenting the ionized analyte into ionized analyte fragments and the ionized analyte-specific internal standard into ionized analyte-specific internal standard fragments by collision with particles of a gas phase;
providing a stream comprising the ionized analyte fragments and the ionized analyte-specific internal standard fragments on a detection mass spectrometer; and
recording with the detection mass spectrometer at least two pre-summed mass-resolved spectra and summing the at least two pre-summed mass-resolved spectra to obtain a final mass-resolved spectrum.

The step of providing of the constant stream of ionized sample molecules may be free of a generation and/or a providing of a pulsed stream of ionized sample molecules.

The step of recording of the pre-summed mass-resolved spectra may comprise a pre-summing of native mass-resolved spectra to obtain the pre-summed mass-resolved spectra, which is performed by a first data processor ; and
the step of summing of the at least two pre-summed mass-resolved spectra to obtain a final mass-resolved spectrum may be performed by a second data processor different from the first data processor, specifically wherein the first data processor may correspond to a data processor comprised by the detection mass spectrometer and the second data processor may correspond to an external data processor; and/or wherein at least one of the at least two pre-summed mass-resolved spectra may be recorded at a frequency between 20kHz and 100Hz, specifically between 5kHz and 100Hz; and/or
wherein the step of recording the at least two pre-summed mass-resolved spectra may be repeatedly performed during a time span of 5s to 1h, specifically during a time span of 15s to 10 minutes and more specifically during a time span of 30s to 2 minutes; and/or
wherein the at least two pre-summed mass-resolved spectra may correspond to 5 to 500 pre-summed mass-resolved spectra which are summed to obtain a final mass-resolved spectrum.

The step of providing of the constant stream of ionized sample molecules and the analyte-specific internal standard may comprise, prior to the ESI and/or nano-ESI, providing of the sample solution as a pre-processed sample solution including separating a serum from a whole blood sample, collecting the analyte from the serum by at least one of the following: magnetobeads-capturing, immunoassay methods, and/or adding the analyte-specific internal standard; and/or
the constant stream of ionized sample molecules and the analyte-specific internal standard and/or the stream of ionized analyte fragments and ionized analyte-specific internal standard fragments may be generated for a time span of up to about 15 minutes, specifically at a pre-processed sample solution volume of 1nL/min to 1000nL/min, specifically 200nL/min to 500nL/min, more specifically 250nL/min to 350nL/min.

The step of summing the at least two pre-summed mass-resolved spectra to obtain the final mass-resolved spectrum may be completed, stopped and/or considered sufficiently sensitive when a coefficient of variance and/or an S/N ratio reaches a pre-defined threshold value, specifically the threshold value corresponds to 20% .

The performing of the first separation may comprise ion mobility spectrometry; and/or
the performing of the second separation may comprise quadrupole mass spectrometry.

The detection mass spectrometer may comprise and/or may be based on at least one of the following: time-of-flight (TOF), Triple Quad (QQQ), Ion Trap, an Orbi-Trap mass spectrometry, sector field MS, specifically wherein the detection mass spectrometer is combined with an ionization unit comprising at least one of the following: NanoSpray, ESI, APCI.

The step of performing the second separation may be repeated at least one time.

The step of providing of the constant stream of the analyte and the analyte-specific internal standard may be performed over a providing time span and the step of performing of the spatial accumulation of the analyte and the analyte-specific internal standard may be performed over an accumulation time span.

The step of performing of the first separation of the analyte and the analyte-specific internal standard may be performed in a pulsed manner over first separation time spans.

The step of performing of the second separation of the analyte and the analyte-specific internal standard may be performed in a pulsed manner over second separation time spans.

The step of fragmenting of the ionized analyte into ionized analyte fragments and the analyte-specific internal standard into ionized analyte-specific internal standard fragments may be performed in a pulsed manner over fragmenting time spans.

The step of recording with the detection mass spectrometer at least two pre-summed mass-resolved spectra may be performed in a pulsed manner over recording time spans , specifically
wherein (t0a) > (t1) > (t2) = (t3) > (t4) or (t0a) > (t1) > (t2) > (t3) > (t4), and/or
wherein the providing time span may range between 15s and 30 minutes, each of the first separation time spans may range between 40ms and 300ms, each of the second separation time spans may range between 1ms and 40ms, each of the fragmenting time spans may range between 1ms and 40ms and each of the recording time spans may be shorter than 1ms, specifically may range between 1ms and 50µs.

The step of providing of the constant stream of the analyte and the analyte-specific internal standard may be performed constantly.

The step of performing of the first separation of the analyte and the analyte-specific internal standard may be performed at a frequency of 25Hz to 3Hz.

The step of performing of the second separation of the analyte and the analyte-specific internal standard may be performed at a frequency of 1kHz to 25Hz.

The step of fragmenting of the ionized analyte into ionized analyte fragments and the analyte-specific internal standard into ionized analyte-specific internal standard fragments may be performed at a frequency of 1kHz to 25Hz.

The step of recording with the detection mass spectrometer of at least two pre-summed mass-resolved spectra may be performed at a frequency exceeding 1kHz.

Below are described some specific optional embodiments and features of the system according to the second aspect or embodiments thereof.

The mass spectrometer system may be suited for detecting at least one analyte in a biological sample solution and may comprise:
a sample supply module, comprising a sample pre-processor and an ionizer, the sample supply module being configured to provide a constant stream of ionized sample molecules including the analyte from a sample solution and an analyte-specific internal standard, wherein the ionizer is based on electrospray ionization (ESI) and/or nano-electrospray ionization (nano-ESI);
a first selectivity enhancer module configured to perform a spatial accumulation of the analyte and the analyte-specific internal standard and to perform at least partially a first separation of the analyte and the analyte-specific internal standard from other components of the ionized sample molecules based on the size and/or shape of the ionized molecules;
a second selectivity enhancer module configured to perform at least partially a second separation of the analyte and the analyte-specific internal standard from remained other components of the ionized sample molecules based on the mass to charge ratio of the ionized molecules;
a fragmenter module configured to fragment the ionized analyte into ionized analyte fragments and the analyte-specific internal standard into ionized analyte-specific internal standard fragments by collision with particles of a gas phase;
a detection module comprising a detection mass spectrometer with a first data processor and being configured to record at least two pre-summed mass-resolved spectra from a stream of the ionized analyte fragments and the ionized analyte-specific internal standard fragments; and a controller module comprising a second data processor configured to sum the at least two pre-summed mass-resolved spectra to obtain a final mass-resolved spectrum.

The sample pre-processor may be configured to collect the analyte molecules from the serum by at least one of the following: magnetobeads-capturing and/or immunoassay methods.

The first selectivity enhancer module may comprise an ion mobility spectrometer. Alternatively, the mass spectrometer may be completely free of an ion mobility spectrometer and all the disclosed features may also be combinable with a mass spectrometer that is free of IMS.

The second selectivity enhancer module and/or the fragmenter module may comprise a quadrupole mass spectrometer and/or a gas-filled chamber.

The mass spectrometer system and/or the mass spectrometer may comprise at least one of the following: time-of-flight (TOF), Triple Quad (QQQ), Ion Trap, an Orbi-Trap mass spectrometry.

The first selectivity enhancer module may provide an effective length configured for the first separation of the analyte and the analyte-specific internal standard from other components of the ionized sample molecules of 3cm to 8m, specifically of 4cm to 3m, more specifically of 5cm or of 1,5m, specifically wherein the first selectivity enhancer module comprises and/or corresponds to a TIMS cartridge with an accumulation tunnel and a TIMS tunnel, wherein the TIMS tunnel provides the effective length configured for the first separation.

### Detailed Description of the Invention

In the following, some example embodiments will be described in detail, wherein the invention should not be understood to be limited to the examples and embodiments described. The following examples and embodiments are provided to aid the understanding of the present invention, the true scope of which is set forth in the appended claims. Single features being described in a particular embodiment may be arbitrarily combined, given that they are not excluding each other. In addition, different features, which are provided together in the example embodiments are not to be considered restrictive to the invention or the embodiment.

### Brief Description of the Figures

**FIG. 1** is a flow chart illustrating a mass spectrometric method for detecting an analyte from a biological sample according to an embodiment;
**FIG. 2** is a flow chart illustrating a mass spectrometric method including steps for providing a suspension and an un-used substrate, as well as a substrate removal step according to an embodiment;
**FIG. 3** is a flow chart illustrating a mass spectrometric method including a sample pre-processing stage with washing steps according to an embodiment;
**FIG. 4** is a flow chart illustrating a mass spectrometric method including sample drying steps according to an embodiment;
**FIG. 5** is a flow chart illustrating a detecting step comprising recording and digitally summing mass-resolved spectra according to an embodiment; and
**FIG. 6** is a schematic drawing of a mass spectrometer system according to an embodiment.

### Detailed Description of the Figures

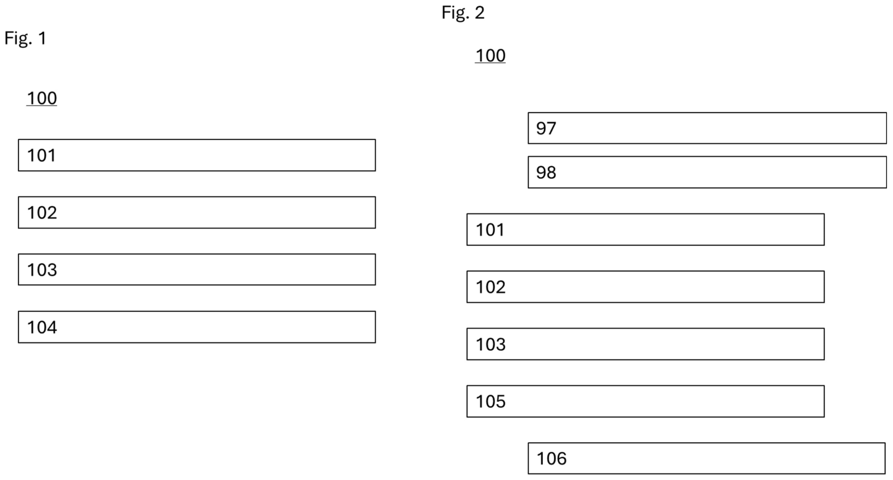

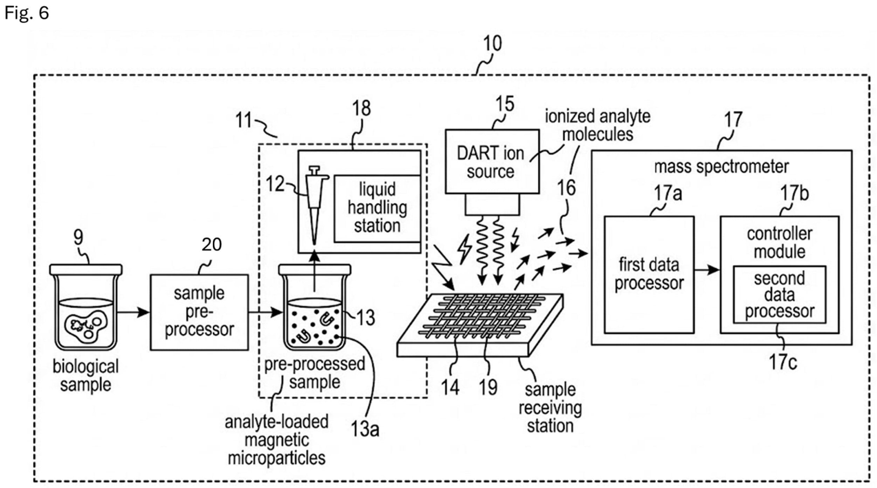

The flowcharts of **Fig. 1** to **Fig. 5** may be understood together with the schematic drawing of **Fig. 6****.**

**FIG. 6** is a schematic drawing of a mass spectrometer system 10 for detecting an analyte from a biological sample 9 according to an embodiment. The system 10 comprises a sample pre-processor 20 configured to process the biological sample 9 to obtain a pre-processed sample 13 containing analyte-loaded magnetic microparticles 13a. A transfer unit 11, which may be embodied as a liquid handling station 18, is configured to collect the pre-processed sample 13 using a transfer device 12, such as a disposable and/or synthetic pipette tip, and transfer it to a substrate 14.

The substrate 14, which may - among other embodiments -be a metallic mesh or grid, is positioned at or on a sample receiving station 19. A Direct Analysis in Real Time (DART) ion source 15 is configured to generate ionized analyte molecules 16 directly from the microparticles on the substrate 14. The mass spectrometer 17 is configured to detect these ionized analyte molecules 16. The mass spectrometer 17 in this embodiment includes a first data processor 17a for recording at least two pre-summed mass-resolved spectra and a controller module 17b comprising a second data processor 17c configured to digitally sum the spectra to obtain a final mass-resolved spectrum. The mass spectrometer 17 may however in other embodiments be free of a controller module 17b and/or a second data processor 17c.

**FIG. 1** is a flow chart illustrating a mass spectrometric method 100 for detecting an analyte from a biological sample 9 according to an embodiment. The method comprises the steps of: collecting 101 with a transfer device 12 a pre-processed sample 13 comprising analyte-loaded magnetic microparticles 13a to which the analyte is captured; transferring 102 with the transfer device 12 the pre-processed sample 13 to a substrate 14; subjecting 103 the pre-processed sample 13 on the substrate 14 to Direct Analysis in Real Time (DART) ionization to generate ionized analyte molecules 16; and detecting 104 the ionized analyte molecules 16 using a mass spectrometer 17.

**FIG. 2** is a flow chart illustrating a specific embodiment of the mass spectrometric method 100. In this embodiment, the method further comprises providing 98 the pre-processed sample 13 as a suspension prior to the step of collecting 101. Moreover, the substrate 14 is provided as an un-used substrate 97 before the transferring step 102. Following the subjecting step 103, the substrate 14 is removed 106 after single-use. The steps 97, 98 and 106 are optional and do not depend on each other. In other words, each one of the steps 97, 98 and 106 can be avoided and each one of the steps 97, 98 and 106 can be combined with other steps described herein.

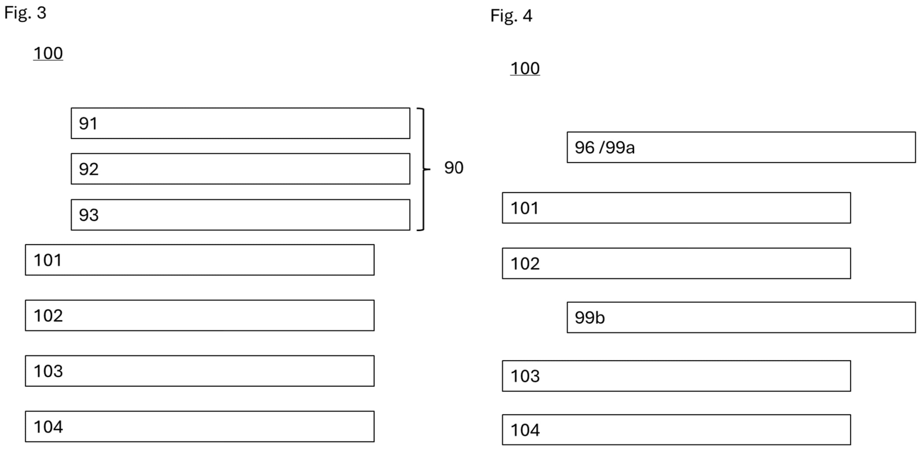

**FIG. 3** is a flow chart illustrating an embodiment of the mass spectrometric method **100** including a sample pre-processing stage **90.** The sample pre-processing **90** comprises providing 91 a mix of the biological sample **9** and functionalized magnetic microparticles, followed by incubating the mix **92** to obtain analyte-loaded magnetic microparticles **13a,** and optionally washing **93** said microparticles to remove interfering components. The enriched analyte-loaded magnetic microparticles **13a** are then subjected to the steps of collecting **101,** transferring **102,** subjecting **103,** and detecting **104.**

**FIG. 4** is a flow chart illustrating an embodiment of the mass spectrometric method **100** incorporating drying steps. The pre-processed sample **13** may be provided in a dried form **96** and/or dried before the collecting step **99a.** Alternatively or additionally, the pre-processed sample **13** is dried on the substrate **99b** after the transferring step **102** but before being subjected **103** to DART ionization.

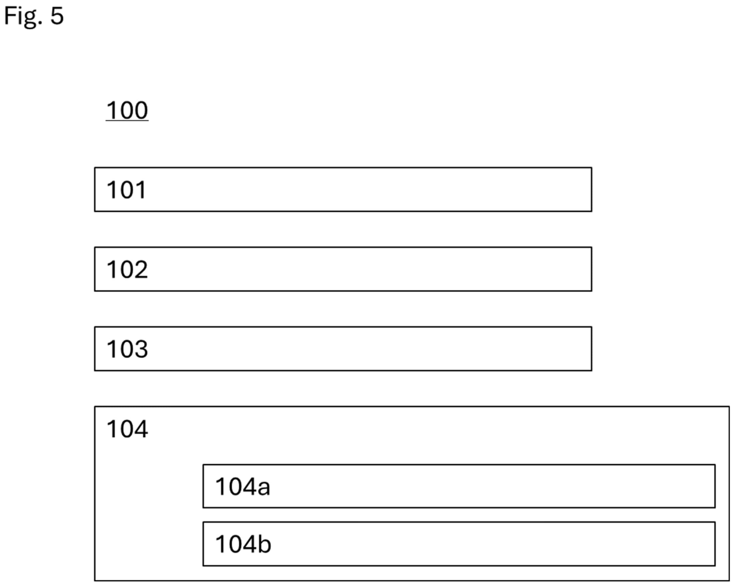

**FIG. 5** is a flow chart illustrating the details of the detecting step **104** according to an embodiment. The step of detecting **104** further comprises recording **104a** at least two pre-summed mass-resolved spectra and digitally summing **104b** these spectra to obtain a final mass-resolved spectrum representing an accumulation of the MS signal.

### Reference Numerals

- 9: biological sample
- 10: mass spectrometer system
- 11: transfer unit
- 12: transfer device
- 13: pre-processed sample
- 13a: analyte-loaded magnetic microparticles
- 14: substrate
- 15: Direct Analysis in Real Time (DART) ion source
- 16: ionized analyte molecules
- 17: mass spectrometer
- 17a: first data processor
- 17b: controller module
- 17c: second data processor
- 18: liquid handling station
- 19: sample receiving station
- 20: sample pre-processor
- 90: sample pre-processing
- 91: providing a mix
- 92: incubating the mix
- 93: washing the analyte-loaded magnetic microparticles
- 96: providing pre-processed sample in dried form
- 97: providing an un-used substrate
- 98: providing suspension
- 99: drying the pre-processed sample
- 99a: drying before collecting
- 99b: drying on the substrate
- 100: mass spectrometric method
- 101: collecting
- 102: transferring
- 103: subjecting
- 104: detecting
- 104a: recording spectra
- 104b: digitally summing
- 106: removing substrate

## Claims

1. A mass spectrometric method (100) for detecting an analyte from a biological sample, the method comprising:
Collecting (101) with a transfer device a pre-processed sample comprising analyte-loaded magnetic microparticles to which the analyte is captured;
Transferring (102) with the transfer device the pre-processed sample to a substrate;
Subjecting (103) the pre-processed sample on the substrate to Direct Analysis in Real Time (DART) ionization to generate ionized analyte molecules;
Detecting (104) the ionized analyte molecules using a mass spectrometer.

2. The mass spectrometric method (100) of claim 1, comprising:
Prior to the step of collecting (101), providing (98) the pre-processed sample comprising a suspension; and
wherein the transfer device comprises a synthetic tip configured to receive at least a portion of the suspension, more specifically a disposable tip and/or a pipette tip.

3. The mass spectrometric method (100) of claim 1 or 2, wherein the substrate comprises: a metallic substrate, specifically a permeable and/or porous metallic substrate, specifically at least one of: a metallic mesh; a metallic grid; a metallic plate with openings.

4. The mass spectrometric method (100) of any one of the preceding claims, further comprising:
Prior to the step of transferring (102) the pre-processed sample to the substrate, a step of providing (97) an un-used substrate; and/or
As a final method step, removing (106) the substrate after single-use.

5. The mass spectrometric method (100) of any one of the preceding claims, further comprising a step of sample pre-processing (90) to obtain the pre-processed sample, specifically wherein the sample pre-processing comprises:
Providing (91) a mix comprising: the biological sample or a derivative thereof comprising the analyte; and the magnetic microparticles that are surface functionalized to selectively bind the analyte from the biological sample or the derivative thereof;
Incubating (92) the mix to obtain the analyte-loaded magnetic microparticles, more specifically wherein the sample pre-processing (90) comprises:
Washing (93) the analyte-loaded magnetic microparticles.

6. The mass spectrometric method (100) of any one of the preceding claims, further comprising:
Prior to the step of collecting (101), providing (96) the pre-processed sample in a dried form; and/or
Drying (99) the pre-processed sample, specifically drying (99a) the pre-processed sample before the step of collecting (101) the pre-processed sample and/or drying (99b) the pre-processed sample on the substrate before the step of subjecting (103) the pre-processed sample to DART ionization (103).

7. The mass spectrometric method (100) of claim 6, wherein, in a case when the pre-processed sample is provided in a dried form prior to the step of collecting (101), the transfer device comprises a synthetic element configured to receive at least a portion of the pre-processed sample in a dried form, more specifically comprising at least one of: a disposable spatula; a disposable spoon; a disposable spatula tip; a disposable spoon tip.

8. The mass spectrometric method (100) of any one of the preceding claims, wherein the substrate is the sole contact point for the microparticles during the DART ionization step.

9. The mass spectrometric method (100) of any one of the preceding claims, wherein the step of detecting (104) further comprises recording (104a) at least two pre-summed mass-resolved spectra and digitally summing (104b) the at least two pre-summed mass-resolved spectra to obtain a final mass-resolved spectrum representing an accumulation of the MS signal.

10. The mass spectrometric method (100) of claim 9, wherein the step of detecting (104) is performed using the mass spectrometer being free of an Ion Mobility Spectrometry (IMS) separation.

11. A mass spectrometer system (10) for detecting an analyte from a biological sample (9), the system comprising:
A transfer unit (11) configured to collect with a transfer device (12) a pre-processed sample (13) comprising analyte-loaded magnetic microparticles (13a) to which the analyte is captured and the transfer unit (11) configured to transfer with the transfer device (12) the pre-processed sample (13) to a substrate (14);
A Direct Analysis in Real Time (DART) ion source (15) configured to generate ionized analyte molecules (16) from the analyte-loaded magnetic microparticles on the substrate (14); and
A mass spectrometer (17) configured to detect the ionized analyte molecules (16).

12. The mass spectrometer system (10) of claim 11, further comprising a sample pre-processor (20) configured to pre-process the biological sample (9) or derivative thereof to obtain the analyte-loaded magnetic microparticles (13a).

13. The mass spectrometer system (10) of claim 11 or 12, wherein the mass spectrometer (17) comprises:
A first data processor (17a) configured to record at least two pre-summed mass-resolved spectra, specifically, wherein the mass spectrometer system (10) further comprises:
A controller module (17b) comprising a second data processor (17c) configured to digitally sum the at least two pre-summed mass-resolved spectra to obtain a final mass-resolved spectrum.

14. The mass spectrometer system (10) of any one of claims 11 to 13, further comprising a liquid handling station (18) equipped with the transfer device (12), the transfer device (12) comprising a disposable and/or synthetic pipette tip configured to aspirate a suspension comprising the analyte-loaded magnetic microparticles and displace said suspension onto the substrate.

15. The mass spectrometer system (10) of any one of claims 11 to 14, further comprising a sample receiving station (19) equipped with the substrate (14), the substrate comprising a metallic substrate, specifically a permeable and/or porous metallic substrate, more specifically at least one of: a metallic mesh; a metallic grid; a metallic plate with openings; or the substrate comprising a non-metallic substrate, specifically a ceramic substrate and/or a glass substrate, more specifically a porous ceramic or glass mesh.
